# EUROPÄISCHE PATENTSCHRIFT

(11) **EP 0 584 721 B1**
(45) Veröffentlichungstag und Bekanntmachung des Hinweises auf die Patenterteilung: **12.11.1997**
(21) Anmeldenummer: 93113265.8
(22) Anmeldetag: 19.08.1993
(51) Int. Cl.: G01N 33/52, B32B 15/04, C30B 25/06

(54) **Analyseelement zur Analyse einer flüssigen Probe**
Analytical element for analysing a liquid specimen
Elément d'analyse pour l'analyse d'un échantillon liquide

(30) Priorität: 21.08.1992 DE 4227665
(43) Veröffentlichungstag der Anmeldung: 02.03.1994
(73) Patentinhaber: BOEHRINGER MANNHEIM GMBH, 68298 Mannheim (DE)
(72) Erfinder: Harttig, Herbert, Dr., D-67067 Ludwigshafen (DE)
(74) Vertreter: Pfeifer, Hans-Peter, Dr., Dr. H.-P. Pfeifer Dr. P. Jany, Patentanwälte

(56) Entgegenhaltungen:
- DE-A- 2 932 973
- DE-A- 3 617 763
- DE-A- 3 701 833

## Beschreibung

Die Erfindung betrifft ein Analyseelement zur Bestimmung eines Analyten in einer flüssigen Probe, welches in mindestens einer Testschicht ein Reagenzsystem enthält, dessen Reaktion mit der Probe zu einer mittels eines Meßlichtstrahls optisch nachweisbaren Veränderung einer Detektionsschicht des Analyseelementes führt. Die optisch nachweisbare Veränderung ist für die Analyse charakteristisch, d.h. die optisch nachweisbare Veränderung ist eine Meßgröße, aus deren Messung das Analyseresultat bestimmt werden kann.

Es sind verschiedene Ausführungsformen solcher Analyseelemente bekannt. Weit verbreitet sind Teststreifen, die auf einer langgestreckten Tragschicht auf Kunststoff eine oder mehrere Testschichten tragen, in denen die Reagenzien eingebettet sind. Bei einem anderen bekannten Typ von Analyseelementen wird ein mehrschichtiger Verbund von filmförmigen Testschichten mit einer Reagenzschicht auf Basis von Gelatine verwendet. Diese Reagenzfilme sind in ähnlicher Weise wie bei einem photographischen Diapositiv von einem Rahmen aus Kunststoff oder Pappe umgeben.

Zur Durchführung einer Analyse wird die Probe auf das Analyseelement aufgebracht. Die Reaktion von Probe und Reagenzsystem führt zu der optisch nachweisbaren Veränderung der Detektionsschicht. Dies kann insbesondere ein Farbumschlag sein, welcher visuell oder mit Hilfe eines Gerätes, meistens reflexionsphotometrisch, ausgewertet werden kann. Die Erfindung richtet sich insbesondere auf Analyseelemente, die zur reflexionsphotometrischen Auswertung bestimmt sind, jedoch kann sie auch für Analyseelemente vorteilhaft sein, bei denen die Reaktion zum Entstehen oder zur Veränderung einer anderen optisch nachweisbaren Meßgröße führt, beispielsweise einer Fluoreszenz oder einer Lumineszenz.

Bei der Entwicklung von Testträgern stellt sich bereits seit langem das Problem, eine optische Sperrwirkung innerhalb des Testschichtverbundes zu realisieren. Von besonderer Bedeutung ist dies bei Analyseelementen, bei denen die Probe auf einer Seite einer Testschicht oder eines Testschichtverbundes zugeführt wird und auf der gegenüberliegenden Seite die optisch nachweisbare Veränderung in der Detektionsschicht stattfindet. Die erste Seite wird nachfolgend als Probenaufgabeseite, die zweite als Detektionsseite bezeichnet. Die Erfindung richtet sich insbesondere auf derartige Analyseelemente, deren wichtigster Vorteil darin besteht, daß die Probe nicht abgewischt oder abgewaschen werden muß, um die Messung der optischen Meßgröße zu ermöglichen. Sie werden deshalb auch als "Non-Wipe-Analyseelemente" bezeichnet.

Die erwähnte optische Sperrwirkung ist bei Non-Wipe-Analyseelementen, aber auch in anderen Anwendungsfällen, erforderlich, um störende Färbungen, die das optisch gemessene Meßergebnis beeinträchtigen, zu eliminieren. Ein besonders wichtiges Beispiel ist die Analyse von Blut ohne vorausgehende aufwendige Abtrennung der die rote Farbe bewirkenden roten Blutkörperchen (Erythrozyten). Häufig ist eine Erythrozytenabtrennschicht vorhanden, die das Durchdringen der Erythrozyten von der Probenaufgabeseite auf die Detektionsseite verhindert. Dies ist jedoch nicht ausreichend, solange der rote Blutfarbstoff von der Detektionsseite her sichtbar bleibt. Es muß deshalb eine optische Sperrschicht (die vielfach auch als Strahlungsblockierungsschicht bezeichnet wird) vorhanden sein.

Das Problem, diese Funktionen in einfacher, kostengünstiger und wirksamer Weise in einem Analyseelement zu realisieren, beschäftigt die Fachwelt bereits seit langem. Verwiesen sei auf die EP-A-0 302 287 und die darin zitierten Druckschriften, wobei hinsichtlich der Strahlungsblockierungsschicht insbesondere auf die US-A-4 069 017 zu verweisen ist.

Bei praktisch gebräuchlichen Analyseelementen wird die Strahlungsblockierung ausschließlich durch Verwendung von Pigment-Pulvern (vor allem TiO₂) erreicht. Strahlungsblockierungsschichten auf Basis pulverförmiger Pigmente sind allerdings insofern problematisch, als selbst bei sehr hoher Konzentration des Pigmentes eine Mindestdicke von mehr als 10 µm erforderlich ist. Außerdem müssen zur Herstellung der Schicht Bindemittel verwendet werden, die die Reaktion oder den Flüssigkeitstransport der Probe durch die Schicht negativ beeinflussen können. Schließlich sind solche Schichten sehr empfindlich gegen mechanische Beanspruchung.

Bekanntlich ist die Eindringtiefe von Licht und den angrenzenden Wellenlängen des elektromagnetischen Spektrums in Metalle extrem gering. Infolgedessen haben Metalle ausgezeichnete strahlungsblockierende Eigenschaften. Es ist deshalb nicht verwunderlich, daß auch bei Analyseelementen bereits der Versuch gemacht wurde, metallische Schichten als optische Sperrschichten einzusetzen. In dem US-Patent 4 255 384 (entsprechend DE-A-29 32 973) wurde bereits vor mehr als zehn Jahren ein Analyseelement beschrieben, bei dem eine metallische Strahlungsblockierungsschicht im Verbund mit den erwähnten Reagenzfilmen auf Basis von Gelatine zum Einsatz kommt. Diese Filme haben die Eigenschaft, daß sich die Probenflüssigkeit in ihnen in lateraler Richtung sehr schlecht ausbreitet. Der Schichtverbund solcher Analyseelemente enthält deswegen stets eine der Reagenzschicht vorgelagerte poröse Ausbreitungsschicht (spreading layer). In der zitierten Literaturstelle wird beschrieben, daß diese Ausbreitungsschicht oder eine andere, keine Reagenzien enthaltende Tragschicht, mit einem Metallfilm versehen werden kann, um eine Strahlungsblockierungsschicht zu erzeugen. Die Beschichtung erfolgt vorzugsweise durch Aufbringen eines in einem Binder dispergierten Metallpulvers. Daneben wird die Vakuumabscheidung des Metalls auf der inerten Tragschicht beschrieben. Obwohl dieser Vorschlag bereits 1980 publiziert wurde, hat er keine praktische Bedeutung erlangt.

Der Erfindung liegt die Aufgabe zugrunde, ein Analyseelement zu schaffen, welches ein extrem geringes Probevolumen erfordert und dennoch eine hohe Genauigkeit der Analyse gewährleistet.

Die Aufgabe wird erfindungsgemäß gelöst, durch ein Analyseelement zur Bestimmung eines Analyten in einer flüssigen Probe, insbesondere einer Körperflüssigkeit, welches in mindestens einer Testschicht ein Reagenzsystem enthält, dessen Reaktion mit der Probe zu einer mittels eines Meßlichtstrahls optisch nachweisbaren für die Analyse charakteristischen Veränderung einer Detektionsschicht führt, bei welchem eine mindestens ein Reagenz des Reagenzsystems enthaltende flüssigkeitsabsorbierende Reagenzschicht eine unmittelbar auf diese aus der Gasphase aufgebrachte Metallisierung in einer solchen Dicke aufweist, daß sie höchstens 50 % des Meßlichtstrahls passieren läßt.

Im Gegensatz zu dem genannten US-Patent 4 255 384 wird bei der vorliegenden Erfindung das Metall also nicht auf eine reagenzienfreie zusätzliche Tragschicht, sondern aus der Gasphase unmittelbar auf eine Reagenzschicht aufgebracht, also eine Schicht, die hochempfindliche makromolekulare Substanzen, wie beispielsweise Enzyme, enthält.

Hierdurch werden große Vorteile erzielt. Die Metallisierung ist extrem dünn (bevorzugt zwischen 40 und 110 nm, maximal bis ca. 1000 nm) und trägt deswegen zur Flüssigkeitsabsorption des Analyseelementes praktisch nichts bei, während bei den vorbekannten Analyseelementen auf Basis von TiO₂ jeweils auch die optische Sperrschicht und bei den aus dem US-Patent 4 255 384 bekannten Elementen jeweils auch die Tragschicht der metallischen Sperrschicht von der Probenflüssigkeit gefüllt werden mußten.

Das Absorptionsvolumen der Testschichten des Analyseelementes ist entscheidend für die bei der Analyse erforderliche Probenmenge. Dadurch, daß erfindungsgemäß für die optische Sperrwirkung keine zusätzliche absorbierende Schicht erforderlich ist, wird eine wesentliche Einsparung an Probenflüssigkeit erreicht.

Bevorzugt ist die metallisierte Reagenzschicht die Detektionsschicht. Bei einem Non-Wipe-Analyseelement ist sie vorzugsweise die einzige Testschicht, die sich auf der von der Probenaufgabeseite abgewandten Seite der Metallisierung (d.h. stromabwärts von dieser) befindet. Dadurch wird das Absorptionsvolumen des Analyseelementes weiter reduziert.

Durch die Metallisierung wird eine ausgezeichnete optische Sperrwirkung erreicht. Störungen durch farbige Komponenten, die sich in Auswerterichtung hinter der Metallisierung befinden, werden vollständig eliminiert. Die Metallisierung gewährleistet eine hohe Remission des Meßlichtstrahls, d.h. mit einer analytfreien Probe (Leerprobe) wird eine sehr hohe Reflexion der Detektionsschicht bestimmt. Infolgedessen ist die Änderung durch die Färbung der Detektionsschicht bei Gegenwart eines Analyten besonders hoch. Dieser hohe "Signalhub" gewährleistet eine gute Meßgenauigkeit bei der reflexionsphotometrischen Auswertung.

Infolge des auf Basis der Erfindung möglichen extrem geringen Probevolumens wird die Genauigkeit des Tests auch insofern verbessert, als die Meßergebnisse weniger stark vom Hämatokrit-Wert einer Blutprobe abhängen. Es wurde festgestellt, daß die mit Analyseelementen bestimmten analytischen Resultate Abweichungen voneinander zeigen, je nachdem, wieviel rote Blutkörperchen das Blut enthält (dieser Anteil wird als Hämatokritwert bezeichnet). Dieser Effekt wird umso geringer, je weniger Blut von den Testschichten des Analyseelementes absorbiert wird.

Weiterhin leistet die Erfindung einen wesentlichen Beitrag dazu, daß die Gewinnung der Blutprobe mit möglichst geringem Schmerz möglich ist. Blutproben für Analyseelemente werden meist durch einen Stich in den Finger gewonnen. Je weniger Blut für das Analyseelement erforderlich ist, desto geringer kann die Einstichtiefe sein. Dies ist vor allem für Patienten, die entsprechende analytische Untersuchungen regelmäßig durchführen müssen, wie beispielsweise Diabetiker, von größter Bedeutung.

Die Metallisierung wird aus der Gasphase aufgebracht, d.h. mit einem Verfahren, bei dem Metallatome, - moleküle oder Cluster im Gaszustand vorliegen und auf der Oberfläche der Reagenzschicht aufgebracht werden. Dünne Metallschichten werden im Vakuum nach unterschiedlichen bekannten Verfahren hergestellt. Beispiele sind das Bedampfungsverfahren und das Sputterverfahren. Beim Bedampfen befindet sich das Substrat, welches metallisiert werden soll, in der Nähe eines offenen Tiegels, in dem das Metall erhitzt wird. Beim Sputterverfahren werden Partikel, vorzugsweise atomare Ionen, die beispielsweise mit Hilfe einer Gasentladung erzeugt wurden, auf eine feste Oberfläche des für die Metallisierung verwendeten Metalls (Sputtertarget) geschossen, wobei atomare oder niedermolekulare Partikel des Metalls frei werden und sich auf dem in der Nähe des Sputtertargets angeordneten Substrat niederschlagen. Ein weiteres bekanntes Verfahren zur Metallisierung aus der Gasphase ist die Cluster-Ionen-Epitaxie.

Bei jedem dieser Verfahren unterliegt das Substrat hohen Belastungen. Beispielsweise wird beim Aufdampfen das Metall auf Temperaturen von etwa 1100°C bis 1300°C erhitzt, wobei sich das Substrat im Hinblick auf eine ausreichende Ausbeute möglichst nah bei dem Verdampfungstiegel befinden soll. Beim Sputtern entstehen durch die Ionisation UV-Strahlen, die zwangsläufig auf das Substrat auftreffen und dieses einer elektromagnetischen Strahlenbelastung aussetzen. Die kinetische Energie der auf der Oberfläche des Substrats auftreffenden Teilchen wird in Wärme umgewandelt. Außerdem wird durch die Bildung der geschlossenen Metallisierungsschicht Kondensationswärme frei.

Im Rahmen der Erfindung wurde überraschenderweise festgestellt, daß die Reagenzien in der Reagenzschicht durch die Metallisierung an ihrer Oberfläche und durch die thermische und mechanische Belastung bei der Vakuumabscheidung des Metalls nicht geschädigt werden. Daß dies eine für die Fachwelt unerwartete Erkenntnis ist, ergibt sich unter anderem aus der zitierten US-Patentschrift 4,255,384. Dort wird an den Strahlungsblockierungsschichten auf Basis von Pigmenten unter anderem kritisiert, daß diese eine Dicke von mindestens 150 µm haben müssen und daß diese große Dicke die Eigenschaften des Tests negativ beeinflußt. Obwohl demzufolge auch bei der US-Patentschrift eine Reduktion der Schichtdicke angestrebt wird, werden ausschließlich Analyseelement-Gestaltungen beschrieben, bei denen eine Metallschicht auf einer reagenzfreien Trägerschicht angebracht ist.

Gemäß einer besonders bevorzugten Ausführungsform sieht die Erfindung bei einem Non-Wipe-Analyseelement vor, daß die Metallisierung der Reagenzschicht der Blutaufgabeseite des Analyseelementes zugewandt ist und die Reagenzschicht derartig mikroporös ist, daß der Analyt in sie eindringt, Erythrozyten jedoch nicht in sie eindringen. Vorzugsweise ist auch dabei die metallisierte Reagenzschicht die Detektionsschicht des Analyseelementes.

Testschichten für Analyseelemente, die eine Porosität haben, bei der Erythrozyten nicht in sie eindringen können, andererseits aber der Analyt in einem für den Ablauf der Reaktion mit dem Reagenzsystem ausreichenden Maß eindringt, sind seit langem bekannt. In dem US-Patent 3 630 957 (erteilt 1971) ist eine Reagenzfilm-Rezeptur beschrieben, die eine wässrige Dispersion natürlicher oder synthetischer Polymere verwendet. Diese trocknet zu einem wasserfesten Film, in welchen Glucose und ähnliche Analyten eindringen. Die roten Blutbestandteile bleiben auf der Filmoberfläche und können (üblicherweise nach etwa einer Minute) abgewischt werden, um den bei der Reaktion erzeugten Farbumschlag visuell oder photometrisch auf der gleichen Seite, auf der die Probe aufgegeben wurde, auszuwerten. Auch mikroporöse Kunststoffmembranen können in ihrer Porengröße auf das genannte Erfordernis eingestellt werden (US-Patent 3 663 374). Ein spezielles Beispiel solcher Membranen sind Phaseninversionsmembranen (europäische Patentschrift 0 154 839).

In der europäischen Patentanmeldung 0 256 806 ist ein Non-Wipe-Analyseelement zur Bestimmung von Glucose in Vollblut beschrieben, bei welchem die Erythrozyten mit Hilfe einer mikroporösen Membran abgetrennt werden sollen. Ein Produkt auf Basis dieser Publikation ist erhältlich. In der Schrift wird jedoch eingeräumt, daß Störungen u.a. durch den Hämatokrit des Blutes verursacht werden. Diese werden meßtechnisch mit Hilfe einer Zwei-Wellenlängenmessung eliminiert. Dadurch ist es zwar möglich, mit Hilfe des auf die Analyseelemente dieser Literaturstelle speziell abgestimmten Meßgerätes die Glucosekonzentration mit ausreichender Genauigkeit zu bestimmen. Eine visuelle Auswertung ist jedoch nicht möglich. Dies stellt einen erheblichen Nachteil dar, weil trotz der weit verbreiteten instrumentellen Auswertung der Analyseelemente eine zumindest zusätzliche visuelle Kontrolle wünschenswert ist.

In der Praxis zur Abtrennung der roten Blutfarbe bewährt haben sich die erwähnten Mikrofilter-Materialien bisher nur bei Analyseelementen, bei denen die Probenzuführung und die Auswertung auf der gleichen Seite der Detektionsschicht erfolgt, wobei es unvermeidlich erforderlich ist, die Detektionsschichtoberfläche vor der Auswertung abzuwischen oder abzuwaschen, damit die Probe die Auswertung nicht stört. Dies gilt vor allem für stark gefärbte Proben wie Blut.

Für Non-Wipe-Analyseelemente konnte sich dagegen die Idee, die roten Blutkörperchen mit Hilfe einer dünnen Filterschicht entsprechender Porosität abzutrennen, in der Praxis nicht durchsetzen. Umfangreiche praktische Anwendung findet die Abtrennung der Erythrozyten mit Hilfe einer Glasfaserschicht, wie sie in dem US-Patent 4 477 575 beschrieben ist. Die Wirksamkeit einer solchen Glasfaserschicht basiert nicht auf einer Siebwirkung. Hierzu wäre die Dichte der üblicherweise verwendeten Glasfaserstrukturen gar nicht eng genug. Soweit bekannt, ist die Funktion der Abtrennung von Erythrozyten mit Glasfasern vielmehr überwiegend auf die Adhäsion der roten Blutkörperchen an der Glasfaseroberfläche zurückzuführen.

Die Erythrozytenabtrennung mit Glasfasern ist jedoch insofern nicht unproblematisch, als sie einerseits ein verhältnismäßig großes Mindest-Probevolumen erfordert, um die Glasfaserschicht, welche ein relativ hohes Saugvolumen haben muß, ausreichend zu füllen. Zum zweiten sind dünne Glasfaserschichten, wie sie zur Herstellung von Blut-Analyseelementen verwendet werden, sehr empfindlich und deswegen nur mit erheblichem Aufwand in den Produktionsprozess, bei dem sehr viele Analyseelemente rationell hergestellt werden, zu integrieren.

Im Rahmen der Erfindung wurde festgestellt, daß ein Non-Wipe-Analyseelement, welches mit einer außerordentlich geringen Blutmenge auskommt, hergestellt werden kann, wenn man die Metallisierung unmittelbar auf die Blutaufgabeseite einer Detektionsschicht aufbringt, die die erwähnten Erythrozytenabtrenneigenschaften hat. Die Detektionsschicht sollte dabei außerordentlich dünn sein. Bevorzugt ist eine Dicke von weniger als 40 µm, wobei Dicken unter 15 µm besonders bevorzugt sind.

Die Erfindung wird im folgenden anhand eines in den Figuren schematisch dargestellten Ausführungsbeispiels näher erläutert. Es zeigen:
- Fig. 1: ein erfindungsgemäßes Analyseelement im Längsschnitt,
- Fig. 2: eine alternative Ausführungsform eines erfindungsgemäßen Analyseelementes in perspektivischer Darstellung,
- Fig. 3: einen Ausschnitt aus Fig. 2 im Schnitt.

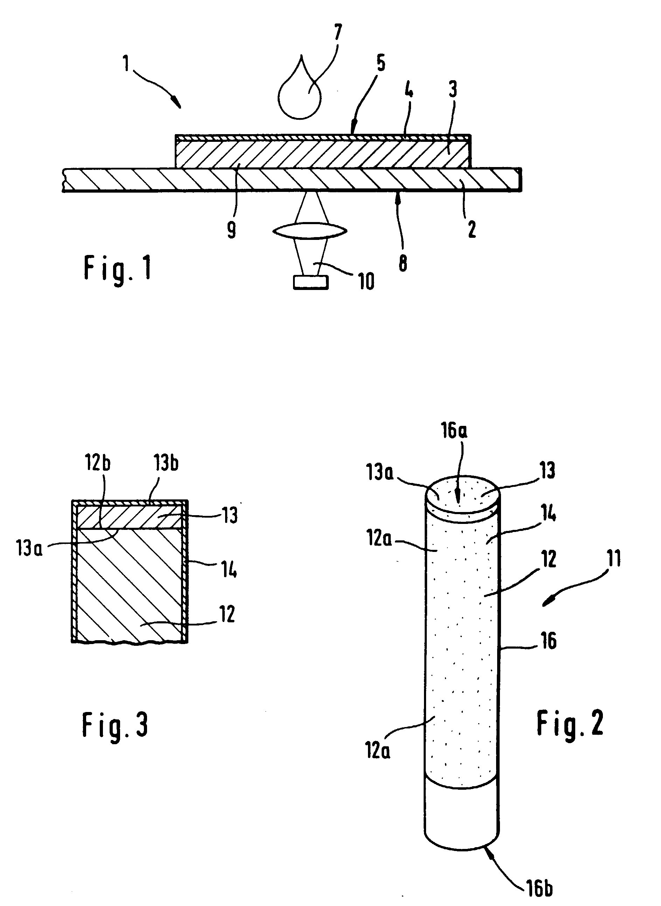

Das in Fig. 1 dargestellte Analyseelement 1 besteht aus einer Basisschicht 2 und einer Reagenzschicht 3, die mit einer Metallisierung 4 versehen ist. Die Metallisierung 4 ist der Probenaufgabeseite 5 zugewandt, auf die eine als Tropfen 7 symbolisierte Blutprobe aufgegeben wird. Die Basisschicht 2 ist der Detektionsseite 8 zugewandt. Die Probe 7 dringt also durch die Metallisierung 4 hindurch in die Reagenzschicht 3 ein. Mit anderen Worten liegt die Reagenzschicht 3 bezogen auf den Flüssigkeitstransportweg der Probe 7 stromabwärts von der Metallisierung 4.

Die im dargestellten bevorzugten Fall einzige Reagenzschicht 3 bildet eine Detektionsschicht 9 des Analyseelementes 1 und enthält sämtliche Reagenzien eines Reagenzsystems zur Bestimmung eines definierten Analyten. Eine aufgrund der Reaktion mit einem darin enthaltenen Reagenzsystem stattfindende optisch nachweisbare Veränderung wird mit einer symbolisch dargestellten Reflexionsmeßeinrichtung 10 gemessen. Dies geschieht üblicherweise mit Hilfe eines speziell auf das Analyseelement 1 abgestimmten Auswertegerätes, welches meist eine mikroprozessorgesteuerte Auswerteeinrichtung einschließt, um den aus der optischen Messung der Detektionsschicht 9 resultierenden optischen Meßwert in das analytische Resultat (meist die Konzentration eines Analyten in der Probe) umzuwandeln. Derartige Auswertegeräte sind bekannt und nicht Gegenstand der vorliegenden Erfindung. Die Erfindung richtet sich jedoch insbesondere auf Analyseelemente, die zur Bestimmung eines Analyten, vor allem Glucose, durch den Patienten selbst ("home-monitoring") dienen, wobei in der Regel kleine, batteriebetriebene tragbare Auswertegeräte eingesetzt werden.

Die Dickenverhältnisse der einzelnen Schichten sind in Fig. 1 nicht maßstäblich und stark übertrieben dargestellt. Üblicherweise hat die Basisschicht 2 eine Dicke von etwa 200 µm, während die Dicke der Reagenzschicht 3 bevorzugt weniger als 40 µm und besonders bevorzugt weniger als 15 µm beträgt. Die Dicke der Metallisierung 4 wird experimentell so festgelegt, daß sie eine ausreichende Strahlungsblockierungswirkung hat. In jedem Fall soll sie nicht mehr als 50 % des Meßlichtstrahls passieren lassen. Bevorzugt ist eine Dicke zwischen 40 und 150 nm, wobei unter Umständen auch stärkere Schichten bis zu etwa 1000 nm verwendet werden können.

Die Basisschicht 2 besteht bevorzugt aus einem durchsichtigen Kunststoffmaterial wie beispielsweise Polycarbonat. In Fällen, bei denen die Reaktion in der Reagenzschicht 3 einen hohen Sauerstoffverbrauch hat, kann es jedoch auch zweckmäßig sein, den Träger für die Reagenzsschicht 3 so zu gestalten, daß ein guter Sauerstoffzutritt möglich ist. Geeignet ist insbesondere ein selbsttragendes Testfeldmaterial auf Basis einer Faserverbundstruktur, wie beispielsweise eines Gewebes oder Gewirkes, bei dem ein Reagenzfilmmaterial in die offenporige Faserverbundstruktur derartig eingebettet ist, daß es deren Poren überspannt und damit schließt. Ein solches Material und ein Verfahren zu seiner Herstellung sind Gegenstand der deutschen Patentanmeldung 42 05 996, auf welche hier Bezug genommen wird.

Im dargestellten bevorzugten Fall ist die Reagenzschicht 3 die einzige Testschicht des Analyseelementes 1. Sie enthält sämtliche Reagenzien des Reagenzsystems. Im allgemeinen Fall kann der Testträger 1 jedoch weitere Testschichten aufweisen, wobei diese vorzugsweise auf der der Probenaufgabeseite 5 zugewandten Seite der Metallisierung 4, also "stromaufwärts" von dieser angeordnet sind. Solche zusätzlichen Testschichten führen zwar zu einer Erhöhung des erforderlichen Probevolumens. Dies muß jedoch in manchen Fällen toleriert werden, z.B. wenn aufgrund von Unverträglichkeiten der Reagenzien, aus herstellungstechnischen Gründen oder weil der Testablauf eine bestimmte Reihenfolge der Kontaktierung der Probe mit den unterschiedlichen Reagenzien erfordert, mindestens eine zusätzliche Testschicht erforderlich ist. Jedenfalls ist bei einem mehrschichtigen Analyseelement die metallisierte Reagenzschicht vorzugsweise die Detektionsschicht und zugleich die im Strömungsweg der Probe letzte Schicht.

Die metallisierte Reagenzschicht 3 enthält vorzugsweise einen polymeren Filmbildner, besonders bevorzugt einen Dispersionsfilmbildner und wird durch Ausstreichen und Trocknenlassen einer filmbildenden Masse ausreichender Viskosität, welche neben dem Filmbildner die Reagenzien enthält, hergestellt. Um das Eindringen der Probe zu fördern, enthält die Reagenzschicht 3 vorzugsweise ein Quellmittel (d.h. eine unter Absorption von Wasser ihr Volumen erhöhende Substanz) und/oder ein wasserlösliches Polymer. Vielfach ist der Zusatz eines Netzmittels (Detergens) zweckmäßig.

Vorzugsweise enthält die Reagenzschicht 3 ein Pigment, insbesondere TiO₂ in einer Konzentration von mindestens 2 %, bezogen auf die Trockenmasse der Reagenzschicht. Im Rahmen der Erfindung wurde festgestellt, daß die Kombination einer pigmenthaltigen Reagenzschicht mit einer zusätzlichen Strahlungsblockierung durch eine Metallisierung zu einer Verbesserung der Analysequalität beiträgt. Dies läßt sich damit erklären, daß die bei der Herstellung der Reagenzschicht unvermeidlichen Dickenschwankungen sich in der Genauigkeit weniger stark auswirken, wenn die Reagenzschicht ein Pigment enthält. Alternativ kann eine Reagenzschicht verwendet werden, die aufgrund ihrer Struktur eine Lichtstreuung bewirkt. Dies gilt insbesondere für mikroporöse Membranen, bei denen die Poren optische Streuzentren bilden.

Die Metallisierung 4 wird wie erwähnt vorzugsweise durch Bedampfen oder durch ein Sputterverfahren aus der Gasphase auf die Reagenzschicht 3 aufgebracht. Zu diesem Zweck wird die Reagenzschicht (zweckmäßigerweise mit einer Tragschicht, auf der sie angebracht ist) in einer Vakuumkammer plaziert. Es können komerziell erhältliche Metallisierungskammern und übliche Verfahrensweisen verwendet werden. Selbstverständlich muß die Reagenzschicht vor der Metallisierung trocken sein. Überraschenderweise hat sich gezeigt, daß keine außergewöhnlichen Maßnahmen ergriffen werden müssen, um eine Beschädigung der Reagenzschicht 3 zu vermeiden. Selbstverständlich sollten jedoch die Verfahrensparameter nicht so gewählt werden, daß sie einer außergewöhnlich hohen Beanspruchung ausgesetzt ist.

Als Materialien für die Metallisierung 4 eignen sich insbesondere Silber und Aluminium. Auch Gold wurde mit Erfolg erprobt, ist jedoch wegen seines hohen Preises und seiner gelben Farbe weniger günstig.

Hinsichtlich der Struktur der Reagenzschichtoberfläche, auf die die Metallisierung 4 aufgebracht wird, ist zu beachten, daß sie ausreichend glatt sein muß, um einen metallischen Glanz der der Reagenzschicht zugewandten Seite der Metallisierung zu gewährleisten. Es wurde festgestellt, daß bei einer stark strukturierten Reagenzschichtoberfläche, die Mikrostrukturen (Erhebungen und Vertiefungen) in der Größenordnung der Lichtwellenlänge aufweist, die Metallisierung schwarz erscheint.

Eine Metallisierung 4 in dem angegebenen Dickenbereich, teilweise auch bis zu höheren Dicken in der Größenordnung von 1 µm verursacht keine durchgreifenden Probleme hinsichtlich der Penetration der Probe in die Reagenzschicht. Die Porosität einer solchen Metallisierung ist so hoch, daß das Eindringen der Flüssigkeit in die Reagenzschicht 3 kaum behindert wird (mit zunehmender Dicke jedoch abnimmt). Aufgrund der Änderung der Oberflächeneigenschaften wurde in einzelnen Fällen sogar beobachtet, daß die Metallisierung 4 zu einer schnelleren Absorption der Probe in der Reagenzschicht 3 beiträgt.

Insgesamt ist es mit dem beschriebenen erfindungsgemäßen Analyseelement 1 möglich, eine extrem kleine Blutprobe (ca. 0,1 µl je Quadratmillimeter Testfläche) in einfacher Weise, schnell und genau auf einen darin enthaltenen Analyten, insbesondere Glucose, zu analysieren. Besonders positiv fällt auf, daß die Meßwerte im Gegensatz zu vorbekannten Non-Wipe-Analyseelementen von dem Hämatokrit-Wert der Blutprobe praktisch vollständig unabhängig sind.

Fig. 2 und Fig. 3 zeigen eine alternative Ausführungsform, bei welcher eine Reagenzschicht 13, die auch hier zugleich die Detektionsschicht 9 ist, auf einer Oberfläche 12b eines Lichtleiters 12 angeordnet ist. Im dargestellten Fall ist der Lichtleiter 12 ein zylinderförmiger Stift 16, dessen erste Stirnseite 16a die Oberfläche 12b bildet, die die Reagenzschicht 13 trägt.

Bei einem solchen Analyseelement wird der Meßlichtstrahl für die Messung der optisch nachweisbaren Veränderung auf diejenige Seite 13a der Reagenzschicht 13 gerichtet, die der Lichtleiteroberfläche 12b benachbart ist, im dargestellten Fall also die dem Stift 16 zugewandte Unterseite 13a der Reagenzschicht 13. Zweckmäßigerweise wird der Meßlichtstrahl durch die zweite Stirnfläche 16b des Stiftes 16 in diesen eingekoppelt. Gemessen wird das von der Reagenzschicht 13 reflektierte Licht.

Derartige Lichtleiter-Analyseelemente sind in verschiedenen Ausführungsformen bekannt. Verwiesen sei insbesondere auf die DE-A-36 17 763 und die DE-A-37 01 833 sowie auf die gleichzeitig eingereichte deutsche Patentanmeldung "Lichtleitendes Analyseelement zur Bestimmung eines Analyts" der gleichen Anmelderin mit dem internen Aktenzeichen "3597/00/DE", auf welche insoweit Bezug genommen wird.

Das bevorzugte Lichtleiter-Analyseelement 11 der vorliegenden Erfindung zeichnet sich dadurch aus, daß eine Metallisierung 14 die von dem Lichtleiter 12 abgewandte zweite Seite 13b der Reagenzschicht 13 und mindestens einen Teil 12a der reagenzschichtfreien Oberfläche des Lichtleiters 12 bedeckt. Hieraus resultieren u.a. folgende Vorteile. Die Metallisierung 14 auf dem reagenzschichtfreien Teil 12a des Lichtleiters 12 verbessert dessen lichtleitende Eigenschaften. Durch die Metallisierung 14 wird eine vollständige Reflexion des in dem Lichtleiter 12 transportierten Lichtes auch dann erreicht, wenn die Totalreflexionsbedingung, auf der die Lichtleitung in einem nichtmetallisierten Lichtleiter basiert, nicht beachtet ist.

Dies wird in einer produktionstechnisch besonders einfachen Art und Weise erreicht. Die Metallisierung 14 kann nämlich in einem Arbeitsgang sowohl auf die Oberfläche 13b der Reagenzschicht 13, als auch auf den reagenzschichtfreien Teil 12a des Lichtleiters 12 aufgebracht werden. Sie erfüllt dabei eine doppelte Funktion einerseits als Strahlungsblockierungsschicht für die Reagenzschicht 13 und andererseits als Reflexionsschicht für den Lichtleiter 12. Von besonderer Wichtigkeit ist dabei, daß die Metallisierung 14 die Reagenzschicht 13 und den daran angrenzenden reagenzschichtfreien Teil 12a des Lichtleiters 12 lückenlos bedeckt und dadurch vollständig gegen Fremdlicht abschirmt. Dies ist bei Lichtleiter-Analyseelementen besonders wichtig, weil eindringendes Fremdlicht deren Auswertung besonders stark stört.

### Beispiel:

Zur Herstellung eines Reagenzfilms wurden zunächst folgende Lösungen hergestellt:

| | | |
|---|---|---|
| a) | 0,129 g | Tetramethylammoniumchlorid |
| | 0,6 g | Phosphormolybdänsäure |
| | 0,75 g | Wasser |
| | | |
| b) | 0,06 g | NN-Bishydroxynitrosoanilin |
| | 3,0 g | Wasser |
| | | |
| c) | 0,586 g | Glucoseoxidase (184 U/mg) |
| | 2,4 g | Wasser |

Die Vorlösungen wurden vermischt mit

| | |
|---|---|
| 2,7 g | einer 2%igen Lösung von Keltrol (Hersteller Kelco/Ail International GmbH, Hamburg, Deutschland) in 0,2 m Citrat-Puffer |
| 0,154 g | einer 15%igen Nonylsulfatlösung |
| 0,3 g | Polyvinylpyrollidon K 25 |
| 3,5 g | Propiofan 70 D (Hersteller: BASF AG, Ludwigshafen, Deutschland) |
| 0,05 g | Tartrazin |
| 0,5 g | Titandioxid E 171 Anatas (Hersteller: Kronos Titan GmbH, Leverkusen, Deutschland) als Dispersion in Wasser (4 Teile Titandioxid auf 5 Teile Wasser) |
| 8,7 g | Wasser |

Darin bilden das Tetramethylammoniumchlorid, die Phosphormolybdänsäure und das Nitrosoanilin in Verbindung mit der Glucoseoxidase ein Reagenzsystem zum Nachweis von Glucose unter reduktiven Bedingungen. Das Propiofan und das Polyvinylpyrollidon sind filmbildende Bestandteile. Das Keltrol ist ein Quellmittel und die Nonylsulfatlösung wirkt als Netzmittel.

Zur Herstellung einer Reagenzschicht zum Nachweis von Glucose wurde die Mischung homogenisiert und mit einem Rakelspalt von ca. 250 µm auf eine transparente Folie aus Polycarbonat aufgerakelt. Nach dem Trocknen resultierte eine Schichtdicke von 30 µm.

Auf die Schicht wurde ein Tropfen Blut mit einem Glucosegehalt von ca. 120 mg/dl appliziert. Bei Betrachtung durch die transparente Polycarbonatfolie hindurch war im Aufgabebereich des Blutstropfens eine bräunlich-rote Verfärbung zu sehen.

Auf eine gemäß der vorstehenden Beschreibung hergestellte Reagenzschicht wurde in einer Sputterkammer MED 010 der Firma Balzers, Liechtenstein, eine Metallisierung aus Silber von 70 nm Dicke aufgebracht. Danach wurde wiederum ein Blutstropfen mit einem Glucosegehalt von 120 mg/dl auf die metallisierte Oberfläche appliziert. Bei Betrachtung durch die transparente Polycarbonatfolie hindurch war im Aufgabebereich des Blutstropfens bereits nach 20 Sek. eine gleichmäßig grünlich-blaue Farbe entsprechend der Indikatorreaktion ohne Störung durch die Rotfärbung des Blutes zu sehen.

## Patentansprüche

1. Analyseelement zur Bestimmung eines Analyten in einer flüssigen Probe (7), insbesondere einer Körperflüssigkeit, welches in mindestens einer Testschicht ein Reagenzsystem enthält, dessen Reaktion mit der Probe (7) zu einer mittels eines Meßlichtstrahls optisch nachweisbaren für die Analyse charakteristischen Veränderung einer Detektionsschicht (9) führt,
bei welchem eine mindestens ein Reagenz des Reagenzsystems enthaltende flüssigkeitsabsorbierende Reagenzschicht (3,13) eine unmittelbar auf diese aus der Gasphase aufgebrachte Metallisierung (4,14) in einer solchen Dicke aufweist, daß sie höchstens 50 % des Meßlichtstrahls passieren läßt.

2. Analyseelement nach Anspruch 1, bei welchem die metallisierte Reagenzschicht (3) die Detektionsschicht (9) ist.

3. Analyseelement nach einem der vorhergehenden Ansprüche, welches eine Blutaufgabeseite (5) und eine Detektionsseite (8) aufweist, wobei auf der Blutaufgabeseite (5) Blut zugeführt und auf der Detektionsseite (8) die für die Analyse charakteristische optisch nachweisbare Veränderung in der Detektionsschicht detektiert wird.

4. Analyseelement nach Anspruch 3, bei welchem die Metallisierung (4) der Reagenzschicht (3) der Blutaufgabeseite (5) zugewandt ist und die Reagenzschicht (3) derartig mikroporös ist, daß der Analyt in sie eindringt, Erythrozyten jedoch nicht in sie eindringen.

5. Analyseelement nach einem der vorhergehenden Ansprüche, bei welchem die metallisierte Reagenzschicht (3) weniger als 40 µm, bevorzugt weniger als 15 µm dick ist.

6. Analyseelement nach einem der Ansprüche 3 bis 5, bei welchem die metallisierte Reagenzschicht (3) die einzige Reagenzschicht des Analyseelementes ist, die sich auf der von der Probenaufgabeseite (5) abgewandten Seite der Metallisierung (4) befindet.

7. Analyseelement nach einem der vorhergehenden Ansprüche, bei welchem die metallisierte Reagenzschicht (3) ein Pigment in einer Konzentration von mindestens 2 % der Trockenmasse enthält.

8. Analyseelement nach einem der vorhergehenden Ansprüche, bei welchem die Reagenzschicht (13) auf einer Oberfläche (12b) eines Lichtleiters (12) angeordnet ist, durch den der Meßlichtstrahl bei der optischen Messung der optisch nachweisbaren Veränderung auf die der Lichtleiteroberfläche (12b) benachbarte erste Seite (13a) der Reagenzschicht (13) gerichtet werden kann und die Metallisierung (14) die zweite Seite (13b) der Reagenzschicht und mindestens einen Teil der nicht von der Reagenzschicht (13) bedeckten Oberfläche des Lichtleiters (12) in der Umgebung der Reagenzschicht (13) bedeckt.

9. Verfahren zur Herstellung einer metallisierten Reagenzschicht für ein Analyseelement nach einem der vorhergehenden Ansprüche, bei welchem die Reagenzschicht in einer Vakuumkammer plaziert und die Metallisierung aus der Gasphase auf mindestens eine Oberfläche der Reagenzschicht aufgebracht wird.

10. Verfahren nach Anspruch 9, bei welchem die metallische Gasphase durch Verdampfen erzeugt wird.

11. Verfahren nach Anspruch 9, bei welchem die metallische Gasphase durch Sputtern erzeugt wird.

## Claims

1. Analysis element for the determination of an analyte in a liquid sample (7), particularly a body fluid, said analysis element containing, in at least one test layer, a reagent system whose reaction with the sample (7) gives rise to a change in a detection layer (9) which is characteristic of the analysis and is optically detectable with the use of a measuring light beam,
in which a fluid-absorbing reagent layer (3, 13) containing at least one reagent of the reagent system has a metal coating (4, 14) applied directly to it from the gaseous phase so thickly that it allows at most 50% of the measuring light beam to pass through.

2. Analysis element according to claim 1, in which the metallised reagent layer (3) is the detection layer (9).

3. Analysis element according to one of the preceding claims, which has a blood application side (5) and a detection side (8), blood being applied on the blood application side (5), and the optically detectable change characteristic of the analysis being detected from the detection side in the detection layer.

4. Analysis element according to claim 3, in which the metal coating (4) of the reagent layer (3) faces the blood application side (5), and the reagent layer is so microporous that the analyte penetrates into it, but the erythrocytes do not penetrate into it.

5. Analysis element according to one of the preceding claims, in which the metallised reagent layer (3) is less than 40 µm thick, and preferably less than 15 µm thick.

6. Analysis element according to one of claims 3 to 5, in which the metallised reagent layer (3) is the only reagent layer of the analysis element present on the side of the metal coating (4) which faces away from the sample application side (5).

7. Analysis element according to one of the preceding claims, in which the metallised reagent layer (3) contains a pigment in a concentration of at least 2% of the dry mass.

8. Analysis element according to one of the preceding claims, in which the reagent layer (13) is disposed on a surface (12b) of a light conductor (12) through which, during the optical measurement of the optically detectable change, the measuring light beam can be directed towards the first side (13a) of the reagent layer (13) adjacent to the light conductor surface (12b), and the metal coating (14) covers, in the vicinity of the reagent layer (13), the second side (13b) of the reagent layer and at least a part of the surface of the light conductor (12) which is not covered by the reagent layer (13).

9. Method for the manufacture of a metallised reagent layer for an analysis element according to one of the preceding claims, in which the reagent layer is placed in a vacuum chamber, and the metal coating is applied from the gaseous phase to at least one surface of the reagent layer.

10. Method according to claim 9, in which the metallic gaseous phase is produced by evaporation.

11. Method according to claim 9, in which the metallic gaseous phase is produced by sputtering.

## Revendications

1. Elément d'analyse pour déterminer un analyte dans un prélèvement liquide (7), en particulier dans un liquide corporel, qui contient dans une couche de test au moins un système de réactifs, dont la réaction avec le prélèvement (7) aboutit à une modification d'une couche de détection (9), caractéristique pour l'analyse, décelable optiquement au moyen d'un rayon lumineux de mesure, dans lequel une couche de réactif (3,13) absorbant les liquides etv contenant au moins un réactif du système de réactifs comporte une métallisation (4,14) déposée directement en phase gazeuse en une épaisseur telle qu'elle ne laisse passer au plus que 50% du rayon lumineux de mesure.

2. Elément d'analyse selon la revendication 1, dans lequel la couche de réactif (3) constitue la couche de détection (9).

3. Elément d'analyse selon l'une quelconque des revendications précédentes, lequel comporte un côté (5) recevant du sang et un côté (8) de détection, dans lequel du sang est introduit sur le côté (5) recevant le sang et la modification caractéristique pour l'analyse, décelable optiquement dans la couche de détection, est détectée sur le côté (8) de détection.

4. Elément d'analyse selon la revendication 3, dans lequel la métallisation (4) de la couche de réactif (3) est tournée vers le côté (5) recevant le sang et la couche de réactif (3) est microporeuse de sorte que l'analyte y pénètre sans que les érythrocytes n'y pénètrent.

5. Elément d'analyse selon l'une quelconque des revendications précédentes, dans lequel la couche de réactif métallisée (3) est d'une épaisseur inférieure à 40 µm, de préférence inférieure à 15 µm.

6. Elément d'analyse selon l'une quelconque des revendications 3 à 5, dans lequel la couche de réactif (3) métallisée constitue l'unique couche de réactif de l'élément d'analyse qui se trouve sur le côté de la métallisation (4) opposé au côté (5) recevant les prélèvements.

7. Elément d'analyse selon l'une quelconque des revendications précédentes, dans lequel la couche de réactif métallisée (3) contient un pigment en une concentration égale à au moins 2% de la matière sèche.

8. Elément d'analyse selon l'une quelconque des revendications précédentes, dans lequel la couche de réactif (13) est disposée sur une surface (12b) d'un guide optique (12) à travers lequel, lors de la mesure optique de la modification décelable optiquement, le rayon lumineux de mesure peut être dirigé sur le premier côté (13a) de la couche de réactif (13) adjacent à la surface (12b) du guide optique, et la métallisation (14) recouvre le deuxième côté (13b) de la couche de réactif et au moins une partie de la surface du guide optique (12) non recouverte de la couche de réactif (13) aux alentours de la couche de réactif (13).

9. Procédé de fabrication d'une couche de réactif métallisée pour un élément d'analyse selon l'une quelconque des revendications précédentes, dans lequel la couche de réactif est placée dans une chambre à vide et le dépôt de métallisation est effectué en phase gazeuse sur au moins une surface de la couche de réactif.

10. Procédé selon la revendication 9, dans lequel la phase gazeuse métallique est produite par vaporisation.

11. Procédé selon la revendication 9, dans lequel la phase gazeuse métallique est produite par pulvérisation cathodique.
